# EUROPÄISCHE PATENTSCHRIFT

(11) **EP 1 470 211 B1**
(45) Veröffentlichungstag und Bekanntmachung des Hinweises auf die Patenterteilung: **27.08.2008**
(21) Anmeldenummer: 03706394.8
(22) Anmeldetag: 28.01.2003
(51) Int. Cl.: C11D 3/02, A61L 2/00, A61L 2/16

(54) **REINIGUNG UND DESINFEKTION CHIRURGISCHER UND MEDIZINISCHER INSTRUMENTE UND GERÄTE**
CLEANING AND DISINFECTION OF SURGICAL AND MEDICAL INSTRUMENTS AND APPLIANCES
NETTOYAGE ET DESINFECTION D'INSTRUMENTS ET D'APPAREILS CHIRURGICAUX ET MEDICAUX

(30) Priorität: 28.01.2002 DE 10203225
(43) Veröffentlichungstag der Anmeldung: 27.10.2004
(73) Patentinhaber: CHEMISCHE FABRIK DR. WEIGERT (GMBH & CO.), 20539 Hamburg (DE)
(72) Erfinder: TIARKS, Petra, 21031 Hamburg (DE); STAFFELDT, Jürgen, 21423 Winsen/Luhe (DE)
(74) Vertreter: Glawe, Delfs, Moll
(86) Internationale Anmeldenummer: PCT/EP2003/000849
(87) Internationale Veröffentlichungsnummer: WO 2003/064580

(56) Entgegenhaltungen:
- EP-A- 0 701 820
- WO-A-01/41896
- DE-A- 19 710 255
- J. DARBORD: "Inactivation of prions" BIOMED & PHARMACOTHER , Bd. 53, 1999, Seiten 34-38, XP002228686
- P.BROWN, R.ROHWER: JOURNAL OF INFECTIOUS DISEASES, Bd. 153, Nr. 6, Juni 1986 (1986-06), Seiten 1145-1148,
- M.BAIER,A.SCHWARZ, M.MIELKE: JOURNAL OF HOSPITAL INFECTION, Bd. 57, 2004, Seiten 80-84,

## Beschreibung

Die Erfindung betrifft das Gebiet der Reinigung und Desinfektion medizinischer und/oder chirurgischer Instrumente und Apparate.

Die Creutzfeldt-Jakob-Krankheit (CJK) ist eine nach heutiger Erkenntnis durch Prionen hervorgerufene Enzephalopathie. Prionen sind infektiöse Eiweißpartikel, die durch übliche Nukleinsäure angreifende Substanzen nicht ohne weiteres destabilisierbar sind und eine hohe Stabilität gegenüber chemischen und physikalischen Einflüssen besitzen. Die Reinigung und Desinfektion möglicherweise mit Prionen verunreinigter medizinischer oder chirurgischer Instrumente und Apparate ist daher problematisch. In der Literatur (Bundesgesundheitsblatt 7/1998, 279-298) wird vorgeschlagen, mit CJK-Material kontaminierte Instrumente mit 1 bis 2 M NaOH für einen Zeitraum von 24 h oder durch Dampfsterilisation für einen Zeitraum von 1 h bei 134°C zu dekontaminieren. Alternativ wird eine Dekontaminierung mit dem hochgiftigen Guanidiniumthiocyanat vorgeschlagen. Diese Dekontaminierungsverfahren sind extrem aufwendig und nicht bei der routinemäßigen Instrumentenaufbereitung durchführbar.

Der Erfindung liegt die Aufgabe zugrunde, eine Möglichkeit zur Reinigung bzw. Desinfektion medizinischer oder chirurgischer Instrumente und Apparate zu schaffen, bei der Prionen mit hinreichender Sicherheit inaktiviert werden. Die Erfindung soll zur routinemäßigen Anwendung insbesondere bei der maschinellen Instrumentenreinigung und -aufbereitung geeignet sein und keine aufwendige separate Dekontaminierung wie im Stand der Technik erfordern.

Gegenstand der Erfindung ist daher die Verwendung eines Reinigungsmittels gemäß Anspruch 1.

Zunächst seien einige im Rahmen der Erfindung verwendete Begriffe erläutert.

Der Begriff Reinigungsmittel bezeichnet jede anwendungsfertige Formulierung, die entweder unmittelbar oder verdünnt mit Wasser zur Reinigung oder Desinfektion der entsprechenden Instrumente Verwendung findet. Im Kontext der Erfindung schließt der Begriff Reinigungsmittel den Begriff Desinfektionsmittel ein. Das Reinigungsmittel kann in fester Form oder vorzugsweise flüssig formuliert sein. Als Reinigungslösung, d.h. anwendungsfertig verdünnt in wäßriger Lösung, weist das Reinigungsmittel einen pH-Wert von 11,5 oder größer auf.

Das erfindungsgemäß verwendete Reinigungsmittel enthält Tenside. Damit werden Verbindungen bezeichnet, welche die Grenzflächenspannung herabsetzen, also amphiphile Verbindungen mit mindestens einem hydrophoben und einem hydrophilen Molekülteil. Im Rahmen der Erfindung sind sämtliche Tenside wie beispielsweise anionische Tenside, nichtionische Tenside, kationische Tenside, amphotere Tenside und Blockcopolymere (insbesondere aus Ethylenoxid- und Propylenoxideinheiten) verwendbar. Beispielhaft verwiesen wird auf Römpp Chemielexikon, 10. Aufl., Stichwort "Tenside".

Die Erfindung findet Einsatz bei der maschinellen und manuellen Reinigung und/der Desinfektion medizinischer und oder chirurgischer Instrumente und/oder Apparate. "Maschinell" bedeutet, daß das Verfahren vorzugsweise in einer Spülmaschine automatisch abläuft und im Zuge der Reinigung bzw. der Desinfektion kein menschlicher Eingriff erforderlich ist. Insbesondere kann erfindungsgemäß in einer üblichen Spül- und Aufbereitungsmaschine für chirurgische Instrumente gearbeitet werden. Besonders bevorzugt wird die Erfindung bei der maschinellen Reinigung und Desinfektion eingesetzt. Sie kann insbesondere zur routinemäßigen, täglichen Instrumentenreinigung verwendet werden.

Die Begriffe "Reinigung und/oder Desinfektion" beinhalten die erforderlichen Schritte bei der Aufarbeitung benutzter Instrumente und Apparate bis hin zum vorzugsweise sterilen Zustand, in dem sie wiederverwendet werden können.

Medizinische und/oder chirurgische Instrumente und Apparate sind sämtliche im medizinischen und Krankenhausbereich eingesetzten Geräte sowie Teile davon, die einer maschinellen Reinigung und Desinfektion grundsätzlich zugänglich sind.

Destabilisieren von Prionen bedeutet, daß ggf. auf der Instrumentenoberfläche anhaftendes infektiöses Prionenmaterial zumindest partiell destabilisiert wird. Bei einer Destabilisierung liegt die pathogene Konformation des Prionenmoleküls nicht mehr vor.

Prioneninaktivierung liegt vor, wenn im Tierversuch festgestellt wird, daß die Infektiosität eines prionenhaltigen Hirnextrakts nach einer Behandlung mit einem zu prüfenden Mittel oder Verfahren nicht mehr vorhanden ist.

Es ist bekannt, daß Prionen im Fettgewebe eingebettet und selbst hydrophob sind und somit für Wasser und wäßrige Lösungen schwer zugänglich sind.

Die Erfindung beruht auf der überraschenden Erkenntnis, daß sich Prionen verhältnismäßig einfach im Rahmen einer routinemäßigen, insbesondere maschinellen Instrumentenreinigung und -aufbereitung im stark alkalischen Milieu destablisieren lassen, wenn gleichzeitig ein Tensid vorhanden ist. Der genaue Wirkmechanismus der erfindungsgemäßen Kombination ist nicht erforscht, jedoch ist davon auszugehen, daß Tenside die Tertiärstruktur der Prionen lockern und so deren Destabilisierung im alkalischen Milieu erleichtern. Der pH-Wert der anwendungsfertig verdünnten Reinigungslösung beträgt wenigstens 11,5, vorzugsweise wenigstens 12, weiter vorzugsweise wenigstens 12,5. Das Reinigungsmittel enthält Alkalihydroxide wie Natriumhydroxid oder bevorzugt Kaliumhydroxid. Die Verwendung von Kaliumhydroxid erleichtert das Bereitstellen eines Reinigungsmittels in Form eines Konzentrats, da Kaliumhydroxidlösungen bei niedrigen Temperaturen weniger zur Auskristallisation neigen als Natriumhydroxidlösungen. Das Reinigungsmittel kann zusätzlich Alkanolamine enthalten.

Durch den Zusatz von Tensiden zu der hochalkalischen Reinigerlösung wird die Oberflächen- und Grenzflächenspannung deutlich reduziert. Es ist davon auszugehen, daß dadurch die Prionen dem Alkaliwirkstoff besser zugänglich gemacht werden und zumindest die Tertiärstruktur der Prionen zerstört und die Prionen destabilisiert bzw. inaktiviert werden können.

Grundsätzlich sind nichtionische Tenside wie bspw. Fettalkohole am besten zur Verminderung der Oberflächenspannung einer wäßrigen Lösung geeignet. Sie haben den zusätzlichen Vorteil, daß sie wenig schäumen und somit die unerwünschte Schaumbildung bei der Reinigung medizinischer Instrumente verhindern oder vermindern. Eine Schaumbildung kann insbesondere die Reinigung bspw. englumiger Schläuche von Endoskopen oder dergleichen beeinträchtigen. Nichtionische Tenside sind in stark alkalischem Milieu jedoch häufig schwierig in Lösung zu bringen. Es ist daher im Rahmen der Erfindung bevorzugt, daß nichtionische Tensid mit kationischen, anionischen oder besonders bevorzugt amphoteren Tensiden zu kombinieren, die für das nichtionische Tensid als Lösungsvermittler wirken können.

Die anwendungsfertig verdünnte Reinigerlösung weist eine Oberflächenspannung von weniger als 50 mN/m, vorzugsweise weniger als 40 mN/m, weiter vorzugsweise weniger als 35 mN/m, weiter vorzugsweise weniger als 30 mN/m auf. Die Oberflächenspannung wird bestimmt nach der sogenannten Bügel-Ring-Methode nach DIN 53993.

Ein weiterer Aspekt der Erfindung ist die Vermeidung oder Verminderung der sogenannten Redeposition prionenhaltiger Verunreinigungen auf den Instrumenten. Der Begriff Redeposition bezeichnet die Wiederablagerung einer bereits von einer kontaminierten Oberfläche entfernten Verunreinigung auf einer anderen, möglicherweise vorher nicht kontaminierten Oberfläche des zu reinigenden Instruments. Die Redeposition ist ein besonderes Problem bei der im Bundesgesundheitsblatt (7/1998, 279-298) empfohlenen Dekontamination durch 24stündiges Einlegen in 1 bis 2 M NaOH.

Bereits die im Rahmen der Erfindung vorgesehene Verwendung von Tensiden verhindert die Redeposition, da die Tenside abgelöste Prionenbestandteile emulgieren können und damit in der wäßrigen Lösung in Schwebe halten. Besonders bevorzugt ist im Rahmen der Erfindung zur Vermeidung oder Verringerung der Redeposition, daß das Reinigungsmittel zusätzlich Härtedispergatoren enthält. Als Härtedispergatoren können bspw. Phosphate und Polyphosphate, Komplex- oder Chelatbildner oder andere sogenannte Builder verwendet werden. Härtedispergatoren unterstützen die emulgierende Wirkung der Tenside und tragen somit zur Verhinderung der Redeposition bei.

Ein wichtiger Aspekt der Erfindung ist deren Eignung zur routinemäßigen, insbesondere maschinellen Instrumentenreinigung und -aufbereitung. Für eine solche routinemäßige Reinigung werden im Stand der Technik üblicherweise schwachsaure oder schwachalkalische (bspw. enzymatische) Reiniger verwendet, da stark alkalische Lösungen zu einer erhöhten Beanspruchung oder Korrosion und damit Verschleiß von verschiedenen Materialien und Oberflächen führen können, die bei medizinischen Instrumenten und Apparaten verwendet werden. Problematisch in dieser Hinsicht sind bspw. Silikonelastomere, verchromte Instrumente, Lötverbindungen aus Silber und Zinn, Klebverbindungen und Dichtungsmaterialien, Kunststoffüberzüge wie bspw. Farbcodierungen, Glasfaserlichtleiter und optische Oberflächen mit Antireflexvergütung. Besonders problematisch sind Aluminiumoberflächen, insbesondere eloxierte Aluminiumoberflächen, da alkalische Lösungen diesen gegenüber eine besondere Aggressivität aufweisen. Die genannte Problematik tritt bspw. besonders auf bei der Reinigung von Endoskopen und deren Bestandteilen, da hier die zu reinigenden Oberflächen eine große Materialvielfalt aufweisen.

Bei einer besonders bevorzugten Ausführungsform der Erfindung enthält daher das Reinigungsmittel zusätzlich Korrosionsinhibitoren. Darunter ist jeder Stoff zu verstehen, der in der alkalischen Lösung deren Angriff auf Oberflächen, insbesondere mit metallischen Oberflächen wie Aluminium oder eloxiertes Aluminium, hemmt. Geeignete Inhibitoren sind bspw. polymere Silicate wie bspw. Wasserglas, Phosphorsäureester oder dergleichen. Geeignete Phosphorsäureester sind Mono- und/oder Diester der Phosphorsäure mit aliphatischen Alkolholen der Kettenlänge C₁ bis C₂₂ und/oder aliphatischen Diolen und/oder aliphatischen Polyolen der Kettenlänge C₂ bis C₂₂. Besonders bevorzugt ist ein Diester der Phosphorsäure mit Butanol einerseits und Ethylenglykol andererseits. Dieser Ester ist kommerziell unter der Bezeichnung Hordaphos® MDGB erhältlich. Erfindungsgemäß erhält man so trotz der Verwendung hochalkalischer Reinigerlösungen eine schonende Einwirkung auf bspw. eloxierte Aluminiumoberflächen.

Erfindungsgemäß wird aus den Bestandteilen des Reinigungsmittels vorzugsweise ein flüssiges Konzentrat formuliert, das mit Wasser zu der anwendungsfertigen Reinigungslösung verdünnt werden kann. In diesem Konzentrat liegt der Alkaligehalt (berechnet als KOH) vorzugsweise zwischen 2 und 30 Gew.-%, weiter vorzugsweise 35 Gew.-%, weiter vorzugsweise 10 und 30 Gew.-%, weiter vorzugsweise 15 und 25 Gew.-%. Der Tensidgehalt liegt bevorzugt zwischen 2 und 25 Gew.-%, weiter vorzugsweise 2 und 15 Gew.-%, weiter vorzugsweise 5 und 15 Gew.-%, weiter vorzugsweise 5 und 10 Gew.-%. Dieses Konzentrat wird vorzugsweise in Konzentrationen von 0,5 bis 5, vorzugsweise 0,5 bis 2, besonders bevorzugt 0,5 bis 1,5 Vol.-% mit Wasser zu einer gebrauchsfertigen Lösung angesetzt.

Wie bereits erwähnt, kann das Konzentrat wenigstens einen Komplexbildner, insbesondere Chelatbildner, enthalten. Die Komplexbildner dienen der Wasserenthärtung und können durch Komplexieren von Erdalkalionen die Reinigungswirkung gegenüber Kalkseifen verbessern. Bei den Komplexbildnern kann es sich um Homo-, Co- oder Terpolymere auf der Basis von Acrylsäure oder deren Alkalisalzen handeln, ferner um Phosphonsäuren bzw. deren Alkalisalze, wie bspw. 1-Hydroxyethan-1,1diphosphonsäure, Aminotrismethylenphosphonsäure, Ethylendiaminotetrakismethylenphosphonsäure, Phosphonobutantricarbonsäure; Weinsäure, Citronensäure und Glukonsäure; ferner Nitrilotriessigsäure oder Ethylendiaminotetraessigsäure bzw. deren Salze.

Das Konzentrat kann Nitrilotriessigsäure und/oder ein Salz dieser Säure, besonders bevorzugt deren Trinatriumsalz, enthalten. Der NTA-Zusatz ist vorteilhaft, wenn das Konzentrat mit stark mineralstoffhaltigem (hartem) Wasser zu einer gebrauchsfertigen Lösung angesetzt werden soll.

Dem Konzentrat können übliche Konservierungsmittel zugesetzt werden, bspw. p-Hydroxybenzoesäure oder deren Methylester, 5-Brom-5-Nitro-1,3-dioxan, Salicylsäure, 2-Naphtyl-m-N-Dimethylthiocarbanilat, 5-Chlor-5-methyl-4-isothiazolin-3-on, 2-Methyl-4-isothiazolin-3-on sowie Gemische der beiden letztgenannten Verbindungen. Ein bevorzugtes Konservierungsmittel ist p-Hydroxybenzoesäure bzw. deren Methylester. Mit Hilfe dieser Konservierungsmittel läßt sich Mikroben- und Pilzbefall des Reinigungsmittelkonzentrats vermeiden.

Bei Bedarf können Konfektionierhilfsmittel (Lösungsvermittler) zugegeben werden wie bspw. Natriumcumolsulfonat, Natriumtoluolsulfonat, Natriumxylolsulfonat, Harnstoff, Glykole, insbesondere Polypropylenglykole und Polyethylenglykole, Methylacetamid und Fettalkohole, wie bzw. Cetylalkohol.

Die Aufzählung möglicher Inhaltsstoffe ist nicht abschließend. Es können zusätzlich bspw. Netzmittel, Emulgatoren, schaumbremsende Mittel oder dergleichen zugesetzt werden. Vorteilhaft ist beispielsweise der Zusatz von N-Acylglutamat als Netzmittel.

Die Einwirkzeit des Reinigungsmittels beträgt erfindungsgemäß 5 bis 30 min, vorzugsweise 10 bis 20 min. Vor und/oder nach der Einwirkung des erfindungsgemäß verwendeten Reinigungsmittels können weitere Vorreinigungs-, Reinigungs-, Nachspül- oder Klarspül- oder Desinfektionsschritte vorgesehen werden. Bevorzugt ist es, zunächst ein Vorspülen zur Entfernung grober Verunreinigungen vorzunehmen, dann eine erfindungsgemäße Reinigung/Desinfektion, gefolgt von einem Nachspülen mit heißem Wasser (93° C) zur Thermodesinfektion und Entfernung von Reinigungsmittelresten.

Die Reinigung wird erfindungsgemäß bei einer Temperatur von 50 bis 60° C durchgeführt.

Bei der maschinellen Reinigung sind besonders bevorzugt Temperaturen von 50 bis 60°C, insbesondere etwa 50°C, und eine Einwirkzeit von 10 bis 20 min, vorzugsweise etwa 10 min. ratur. Bei der manuellen Reinigung wird bevorzugt eine höhere, vorzugsweise etwa doppelt so hohe Konzentration des Reinigers wie bei der maschinellen Reinigung eingesetzt. Bspw. wird das Reinigerkonzentrat gemäß dem nachfolgenden Beispiel 1 im Rahmen der maschinellen Reinigung bevorzugt in einer Anwendungskonzentration von etwa 0,5 Vol.-% eingesetzt, bei der manuellen Reinigung in einer Konzentration von 1 Vol.-%.

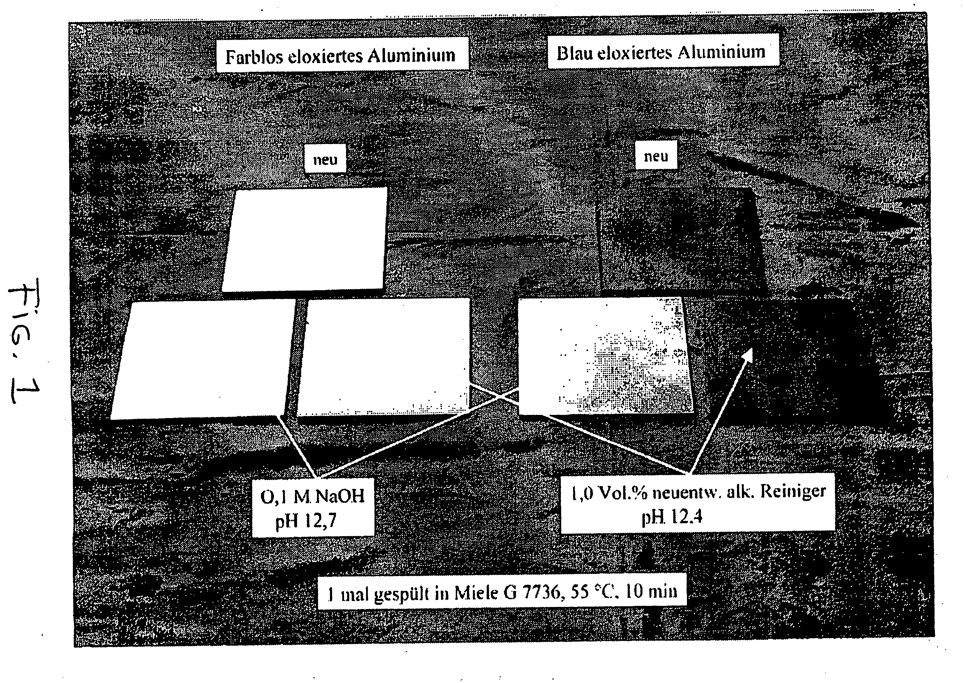

Ausführungsbeispiele der Erfindung werden nachfolgend anhand der Zeichnung und den Beispielen beschrieben.

Die Zeichnung zeigt eloxierte Aluminiumplatten vor und nach der Behandlung mit zwei unterschiedlichen hochalkalischen Reinigern.

### Beispiel 1

Ein Reinigungsmittelkonzentrat wird gemäß der nachfolgenden Tabelle zubereitet. Die Mengen der einzusetzenden Ausgangsstoffe sind in Gewichtsteilen angegeben.

| | |
|---|---|
| Kaliumtripolyphosphat, 50% | 42,78 |
| Kaliumhydroxid, 45% | 22,32 |
| Natriumalkylaminodipropionat | 6,00 |
| Bardac LF¹ | 0,50 |
| Fettalkohol, C10/12, 4EO, 4-5PO² | 0,50 |
| Natriumwasserglas | 27,90 |

| | |
|---|---|
| ¹ Kationisches Tensid (Dioctyldimethyammoniumchlorid) ² Block-Co-Polymer aus C10/C12-Fettalkoholen mit 4 Ethylenoxid- und **4-5** Propylenoxideinheiten. | |

### Beispiel 2

In einer Eintank-Spülmaschine für medizinische und chirurgische Instrumente werden die zu reinigenden Instrumente, bei denen ein Verdacht auf Verunreinigung mit Prionen besteht, zunächst mit Kaltwasser vorgespült. Anschließend wird die Spülmaschine mit kaltem Wasser gefüllt und das Reinigungsmittelkonzentrat gemäß Beispiel 1 in einer Konzentration von 0,5 vol.-% zudosiert. Die Reinigungslösung wird auf 55° C aufgeheizt und 10 min lang bei dieser Temperatur unter Besprühung der Instrumente umgewälzt. Anschließend wird mit kaltem VE-Wasser nachgespült. Zum Schluß erfolgt eine Thermodesinfektion mit VE-Wasser bei 93° C. Diese Thermodesinfektion stellt gleichzeitig die Schlußspülung dar.

### Beispiel 3

Die Oberflächenspannung wurde gemäß DIN 53993 für nachfolgende Flüssigkeiten bestimmt:

| | |
|---|---|
| vollentsalztes (VE) Wasser | 73 mN/m |
| 0,1 N NaOH in VE-Wasser | 72 mN/m |
| 1 Vol.-% Reinigerlösung gemäß | |
| Beispiel 1 in Hamburger Stadtwasser | 33 mN/m |

Man erkennt, daß die Reinigerlösung aus einem Konzentrat gemäß Beispiel 1 eine deutlich verminderte Oberflächenspannung gegenüber einer lediglich alkalischen Lösung aufweist. Diese Lösung enthält die Fettalkohol Verbindung als nichtionisches Tensid sowie Natriumalkylaminodiproprionat als lösungsvermittelndes amphoteres Tensid.

### Beispiel 4

Aufgerauhte Objektträger werden mit 50 mg eines Blut-Eigelb-Gemisches angeschmutzt, bei 55°C 2 h getrocknet und anschließend für 10 min bei Raumtemperatur in ein Rührbad getaucht. Als Medium für das Tauchbad wird VE-Wasser, 0,1 N NaOH oder eine 1 vol.-%ige Reinigerlösung des Reinigungsmittelkonzentrats gemäß Beispiel 1 verwendet. Die anschließend ermittelte Restmenge der Anschmutzung beträgt 20 Gew.-% für Wasser, 35 Gew.-% für 0,1 N NaOH und weniger als 5 Gew.-% für den Reiniger als verwendetes Tauchbadmedium.

### Beispiel 5

Zur Prüfung der Materialschonung eloxierter Aluminiumoberflächen werden eloxierte Aluminiumplatten in der Spülmaschine G7736 der Firma Miele für 10 min bei 55°C einem Reinigungsmedium ausgesetzt. Es werden sowohl neue farblos als auch blau eloxierte Aluminiumplatten verwendet. Das Reinigungsmedium ist 0,1 M NaOH mit einem pH-Wert von 12,7 bzw. eine 1 vol.-%ige Reinigerlösung des Reinigungsmittelkonzentrats gemäß Beispiel 1. Anschließend werden die Platten visuell untersucht. Das in der Zeichnung dargestellte Ergebnis zeigt, daß bei den mit NaOH behandelten Platten die Eloxalschicht deutlich abgetragen ist. Im Unterschied dazu weisen die mit dem Reiniger behandelten Platten keine sichtbaren Schädigungen der Eloxalschicht auf.

## Patentansprüche

1. Verwendung eines Reinigungsmittels, das Tenside und Alkalihydroxid enthält und anwendungsfertig verdünnt in wässriger Lösung einen pH-Wert von wenigstens 11,5 und eine Oberflächenspannung von weniger als 50 mN/m aufweist, zur Inaktivierung von Prionen bei der maschinellen oder manuellen Reinigung und/oder Desinfektion von medizinischen und/oder chirurgischen Instrumenten und Apparaten, wobei die Einwirkzeit des Reinigungsmittels 5 bis 30 min beträgt, und wobei die Reinigung bei einer Temperatur von 50 bis 60°C stattfindet.

2. Verwendung nach Anspruch 1 , **dadurch gekennzeichnet, dass** die Reinigung und/oder Desinfektion maschinell durchgeführt wird.

3. Verwendung nach Anspruch 1 oder 2, **dadurch gekennzeichnet, dass** der pH-Wert wenigstens 12, vorzugsweise wenigstens 12,5 beträgt.

4. Verwendung nach einem der Ansprüche 1 bis 3, **dadurch gekennzeichnet, dass** als Alkalihydroxid KOH verwendet wird.

5. Verwendung nach einem der Ansprüche 1 bis 4, **dadurch gekennzeichnet, dass** das Reinigungsmittel Alkanolamine enthält.

6. Verwendung nach einem der Ansprüche 1 bis 5, **dadurch gekennzeichnet, dass** das Reinigungsmittel nichtionische Tenside enthält.

7. Verwendung nach einem der Ansprüche 1 bis 6, **dadurch gekennzeichnet, dass** das Reinigungsmittel Härtedispergiermittel enthält.

8. Verwendung nach einem der Ansprüche 1 bis 7, **dadurch gekennzeichnet, dass** das Reinigungsmittel anwendungsfertig verdünnt eine Oberflächenspannung von weniger als 40 mN/m, weiter vorzugsweise weniger als 35 mN/m, weiter vorzugsweise weniger als 30 mN/m aufweist.

9. Verwendung nach Anspruch 7, **dadurch gekennzeichnet, dass** das Reinigungsmittel Phosphate und/oder Polyphosphate enthält.

10. Verwendung nach einem der Ansprüche 1 bis 9, **dadurch gekennzeichnet, dass** das Reinigungsmittel Korrosionsinhibitoren enthält.

11. Verwendung nach Anspruch 10, **dadurch gekennzeichnet, dass** die Korrosionsinhibitoren ausgewählt sind aus der Gruppe bestehend aus polymeren Silicaten und Phosphorsäureestern.

12. Verwendung nach einem der Ansprüche 1 bis 11, **dadurch gekennzeichnet, dass** die Einwirkzeit des Reinigungsmittels 10 bis 20 min beträgt.

## Claims

1. The use of a cleaning composition which comprises surfactants and alkali metal hydroxide and has a pH of at least 11.5 and a surface tension of less than 50 mN/m when diluted in an aqueous solution in ready-to-use form, for inactivating prions in the mechanical or manual cleaning and/or disinfection of medical and/or surgical instruments and apparatuses, **characterized in that** the time of action of the cleaning composition is 5 to 30 min and wherein the cleaning takes place at a temperature from 50 to 60°C.

2. The use as claimed in claim 1, **characterized in that** the cleaning and/or disinfection are carried out mechanically.

3. The use as claimed in claim 1 or 2, **characterized in that** the pH is at least 12, preferably at least 12.5.

4. The use as claimed in one of claims 1 to 3, **characterized in that** the alkali metal hydroxide is KOH.

5. The use as claimed in one of claims 1 to 4, **characterized in that** the cleaning composition comprises alkanolamines.

6. The use as claimed in one of claims 1 to 5, **characterized in that** the cleaning composition comprises nonionic surfactants.

7. The use as claimed in one of claims 1 to 6, **characterized in that** the cleaning composition comprises hardness dispersants.

8. The use as claimed in one of claims 1 to 7, **characterized in that** the cleaning composition, diluted in ready-to-use form, has a surface tension of less than 40 mN/m, preferably less than 35 mN/m, further preferably less than 30 mN/m.

9. The use as claimed in claim 7, **characterized in that** the cleaning composition comprises phosphates and/or polyphosphates.

10. The use as claimed in one of claims 1 to 9, **characterized in that** the cleaning composition comprises corrosion inhibitors.

11. The use as claimed in claim 10, **characterized in that** the corrosion inhibitors are selected from the group consisting of polymeric silicates and phosphoric acid esters.

12. The use as claimed in one of claims 1 to 11, **characterized in that** the time of action of the cleaning composition is 10 to 20 min.

## Revendications

1. Utilisation d'un produit de nettoyage qui contient des agents tensioactifs et un alcali caustique et qui, à l'état prêt à l'utilisation, diluée dans une solution aqueuse, possède une valeur pH inférieure à 11,5 et une tension superficielle inférieure à 50 mN/m, destinée à inactiver les prions lors du nettoyage et/ou de la désinfection automatisée ou manuelle d'instruments et d'appareils médicaux et/ou chirurgicaux, le temps d'action du produit de nettoyage étant de 5 à 30 min et le nettoyage étant effectué à une température de 50 à 60°C.

2. Utilisation selon la revendication 1, **caractérisée en ce que** le nettoyage et/ou la désinfection sont effectués de manière automatisée.

3. Utilisation selon l'une quelconque des revendications 1 ou 2, **caractérisée en ce que** la valeur pH est au moins égale à 12, de préférence au moins égale à 12,5.

4. Utilisation selon l'une quelconque des revendications 1 à 3, **caractérisée en ce que** l'alcali caustique utilisé est le KOH.

5. Utilisation selon l'une quelconque des revendications 1 à 4, **caractérisée en ce que** le produit de nettoyage contient des alcanolamines.

6. Utilisation selon l'une quelconque des revendications 1 à 5, **caractérisée en ce que** le produit de nettoyage contient des agents tensioactifs non ioniques.

7. Utilisation selon l'une quelconque des revendications 1 à 6, **caractérisée en ce que** le produit de nettoyage contient des agents de dispersion de dureté.

8. Utilisation selon l'une quelconque des revendications 1 à 7, **caractérisée en ce que** le produit de nettoyage à l'état dilué prêt à l'emploi possède une tension superficielle inférieure à 40 mN/m, encore mieux inférieure à 35 mN/m, encore mieux inférieure à 30 mN/m.

9. Utilisation selon la revendication 7, **caractérisée en ce que** le produit de nettoyage contient des phosphates et/ou des polyphosphates.

10. Utilisation selon l'une quelconque des revendications 1 à 9, **caractérisée en ce que** le produit de nettoyage contient des inhibiteurs de corrosion.

11. Utilisation selon la revendication 10, **caractérisée en ce que** les inhibiteurs de corrosion sont choisis parmi le groupe formé par des silicates polymères et des esters phosphoriques.

12. Utilisation selon l'une quelconque des revendications 1 à 11, **caractérisée en ce que** le temps d'action du produit de nettoyage est de l'ordre de 10 à 20 min.
